# EUROPÄISCHE PATENTSCHRIFT

(11) **EP 2 934 360 B1**
(45) Veröffentlichungstag und Bekanntmachung des Hinweises auf die Patenterteilung: **16.11.2016**
(21) Anmeldenummer: 13817893.4
(22) Anmeldetag: 03.12.2013
(51) Int. Cl.: A61B 90/50, F16D 1/12

(54) **MEDIZINISCHER HALTEARM**
MEDICAL HOLDING ARM
BRAS DE SUPPORT MÉDICAL

(30) Priorität: 20.12.2012 DE 102012112716
(43) Veröffentlichungstag der Anmeldung: 28.10.2015
(73) Patentinhaber: Maquet GmbH, 76437 Rastatt (DE)
(72) Erfinder: WYSLUCHA, Ulrich, 76356 Weingarten (DE); PETER, Stefan, 76437 Rastatt (DE)
(74) Vertreter: Zacco GmbH
(86) Internationale Anmeldenummer: PCT/EP2013/075337
(87) Internationale Veröffentlichungsnummer: WO 2014/095338

(56) Entgegenhaltungen:
- EP-A1- 1 826 301
- DE-A1- 10 209 209
- US-A- 3 419 295
- US-A1- 2009 036 890
- US-B1- 6 767 153

## Beschreibung

Die Erfindung betrifft einen medizinischen Haltearm nach dem Oberbegriff des Anspruchs 1.

Für chirurgische Anwendungen kommen heutzutage vermehrt Assistenzsysteme zum Einsatz die einen Haltearm beinhalten, der beispielsweise an der Gleitschiene eine OP-Tisches anbringbar ist. Ein solcher Haltearm wird beispielsweise bei Schulteroperationen zur Armlagerung eingesetzt. Er weist mehrere starre Halteglieder auf, die über Gelenke beweglich miteinander gekoppelt sind. Dadurch lässt sich der Haltearm frei in allen Raumrichtungen bewegen und sicher in der gewünschten Position fixieren.

Die Gelenke, die jeweils zwei starre Halteglieder des Haltearms frei beweglich miteinander koppeln, weisen einen ersten Gelenkkörper, der mit dem einen Halteglied, und eine zweiten Gelenkkörper auf, der mit dem anderen Halteglied verbunden ist. Um die beiden Halteglieder in einer gewünschten Anordnung zueinander zu fixieren, müssen die beiden Gelenkkörper gegeneinander verriegelt werden.

Eine Möglichkeit für eine solche Verriegelung ist in der DE 102 09 209 B4 beschrieben. Dort weist einer der Gelenkkörper mehrere Raststifte auf, während an dem anderen Gelenkkörper entsprechende Rastvertiefungen ausgebildet sind. Ein wesentliches Merkmal dieses Verriegelungsmechanismus besteht darin, dass die Zahl an Rastvertiefungen größer als die Zahl an Raststiften ist. In einer konkreten Ausführungsform sind dreizehn Rastvertiefungen und zwölf Raststifte vorgesehen. Im verriegelten Zustand greift jeweils einer der Raststifte vollständig in eine der Rastvertiefungen ein, während die diesem Stift unmittelbar benachbarten Raststifte nur teilweise in die ihnen zugeordneten Rastvertiefungen eintauchen. Dadurch wird die spielfreie Verrastung des Gelenks unterstützt.

Um diese Art des Ineinandergreifens der Raststifte in die Rastvertiefungen zu ermöglichen, weisen die Raststifte jeweils einen Eingriffsteil auf, der die Form eines Kegelstumpfs hat. Die Rastvertiefungen sind entsprechend kegelstumpfförmig verjüngt. Dadurch ist sichergestellt, dass im verriegelten Zustand des Gelenks derjenige Raststift, der vollständig in die zugehörige Rastvertiefung taucht, mit seinem Eingriffsteil vollflächig in der Rastvertiefung anliegt. Demgegenüber haben die Eingriffsteile derjenigen Raststifte, die nur teilweise in die den zugehörigen Rastvertiefungen tauchen, lediglich einen Linienkontakt oder zumindest einen sehr geringen Flächenkontakt mit den zugehörigen Rastvertiefungen.

In der praktischen Anwendung hat sicher herausgestellt, dass ein Haltearm, der mit einer Verriegelung vorstehend erläuterter Art arbeitet, im Hinblick auf hohe Lastanwendungen gewissen Beschränkungen unterliegt. So muss der Haltearm mittels eines Handgriffs, der mit einer auf die Gelenke wirkenden Entriegelungsmechanik gekoppelt ist, betätigt werden, um die Gelenke zu entriegeln. Je größer die Last ist, desto mehr Handkraft muss ausgeübt werden.

Aufgabe der Erfindung ist es, ein Haltearm vorstehend beschriebener Art so weiterzubilden, dass höhere Lastanwendungen vom Benutzer bequem gehandhabt werden können.

Die Erfindung löst diese Aufgabe durch einen Haltearm mit den Merkmalen des Anspruchs 1.

Erfindungsgemäß hat der Eingriffsteil des jeweiligen Raststiftes mindestens eine erste abgeflachte Anlagefläche. Entsprechend hat auch die jeweilige Rastvertiefung mindestens eine zweite abgeflachte Anlagefläche. Bei Aufnahme des Eingriffsteils des Raststifts in der Rastvertiefung sind die erste und die zweite Anlagefläche zumindest zum Teil in flächigem Kontakt miteinander.

Unter der erfindungsgemäßen Abflachung der ersten bzw. der zweiten Anlagefläche ist zu verstehen, dass die jeweilige Anlagefläche zumindest weniger stark gewölbt ist als die restliche, vorzugsweise konische Oberfläche des Eingriffsteils des Raststifts bzw. der Rastvertiefung. Dadurch ist insbesondere auch dann, wenn der Eingriffsteil des jeweiligen Raststifts nur teilweise in die zugehörige Rastvertiefung taucht, im Bereich der beiden Anlageflächen für einen flächigen Kontakt zwischen Raststift und Rastvertiefung gesorgt ist. Dadurch sind höhere Lastanwendungen möglich als bei dem in der DE 102 09 209 B4 beschriebenen Verriegelungsmechanismus, der bei nicht vollständigem Eingriff infolge der kegelstumpfförmigen Ausbildung des Raststifts und der Rastvertiefung nur einen annähernd linienförmigen Kontakt zwischen Raststift und Rastvertiefung ermöglicht.

Durch die erfindungsgemäße Formgebung wird also erreicht, dass die im Eingriff befindlichen Komponenten, nämlich der Eingriffsteil des Raststifts und die Rastvertiefung, stets, also insbesondere auch bei geringer Eintauchtiefe, einen flächigen Kontakt miteinander haben, wodurch die Flächenpressung zwischen diesen Komponenten reduziert wird. Infolge des geringeren Losbrechmoments, das durch die geringere elastische Verformung und das Fehlen von Kaltverschweißungseffekten bedingt ist, lässt sich das verriegelte Gelenk mit wesentlich geringerer Handkraft als bisher lösen. Besonders positiv wirkt sich dies bei nur teilweise in die Rastvertiefung eingreifendem Raststift aus. Außerdem ergibt sich eine höhere Verschleißfestigkeit an den Kanten des jeweiligen Raststifts.

Die höhere Belastungsfähigkeit macht den erfindungsgemäßen Haltearm für andere Anwendungen als bisher einsetzbar, insbesondere in hochbelasteten und dynamischen Anwendungsbereichen, z.B. Beinanwendungen, Körperstützen und dergleichen. Auch ist der Haltearm nun auch gegenüber Impulsbelastungen unempfindlich, wie sie beispielsweise beim Einschlagen von Prothesen auftreten.

Vorzugsweise sind die erste und die zweite Anlagefläche jeweils aus einer ebenen Fläche gebildet. Solch ebene Anlageflächen ermöglichen besonders zuverlässig den gewünschten flächigen Kontakt eines nicht vollständig in die zugehörige Rastvertiefung eingreifenden Raststifts, um so die Flächenpressung zu verringern. Es ist jedoch darauf hinzuweisen, dass die Anlageflächen zur Verringerung des Flächenpressung nicht streng eben geformt sein müssen. Auch leicht gewölbte Anlageflächen kommen in Betracht, sofern sie geeignet sind, für den gewünschten flächigen Kontakt zu sorgen.

In einer besonders bevorzugten Ausgestaltung ist die jeweilige Rastvertiefung als Pyramidenstumpf geformt, wobei die zweite Anlagefläche aus einer Seitenfläche der so geformten Rastvertiefung gebildet ist. Der Pyramidenstumpf weist dabei beispielsweise eine rechteckige Grundfläche auf. Diese Ausgestaltung ist jedoch nur beispielhaft zu verstehen. So kann die jeweilige Rastvertiefung auch eine andere Form aufweisen, z.B. die Form eines Langlochs mit zwei einander gegenüberliegenden parallelen Seitenflächen und zwei einander gegenüberliegenden schräg zulaufenden Seitenflächen.

In einer besonders bevorzugten Ausführung ist der Pyramidenstumpf so groß bemessen, dass der Eingriffsteil des jeweiligen Raststifts bei vollständigem Eingriff nur im Bereich seiner Anlagefläche in Kontakt mit der Rastvertiefung ist. So wird jeder Linienkontakt zwischen Raststift und Rastvertiefung, der eine nachteilig hohe Flächenpressung verursacht, vermieden.

Vorzugsweise beinhaltet die mindestens eine erste Anlagefläche des jeweiligen Raststifts mindestens zwei Anlageflächen, die auf diametral entgegengesetzten Seiten des Eingriffsteils des Raststifts angeordnet sind. In diesem Fall weist auch die jeweilige Rastvertiefung vorzugsweise zwei diametral angeordnete zweite Anlageflächen auf. In Abhängigkeit der Drehstellung der beiden Gelenkplatten ist auf diese Weise dafür gesorgt, dass sich stets eine der beiden ersten Anlageflächen in Kontakt mit einer der beiden zweiten Anlageflächen befindet.

Vorzugsweise hat der jeweilige Raststift bei Aufnahme seines Eingriffsteils in der jeweiligen Rastvertiefung nur im Bereich seiner eigens hierfür vorgesehenen Anlagefläche Kontakt mit der Rastvertiefung, nämlich mit deren zweiter Anlagefläche. Diese Vermeidung eines Kontaktes zwischen Raststift und Rastvertiefung außerhalb der beiden Anlageflächen verhindert wiederum jeden unerwünschten Linienkontakt zwischen Raststift und Rastvertiefung und macht das Gelenk unempfindlich gegenüber Fertigungstoleranzen, Gelenkspiel und elastischen Lageänderungen unter Last.

In einer bevorzugten Ausführung bilden die Raststifte und die Rastvertiefungen jeweils eine kreisförmige Anordnung. Vorzugsweise sind dann die ersten Anlageflächen der Raststifte und die zweiten Anlageflächen der Rastvertiefungen jeweils längs dieser kreisförmigen Anordnung ausgerichtet. Dies bedeutet, dass sowohl die ersten Anlageflächen als auch die zweiten Anlageflächen jeweils von einer imaginären Kreislinie durchsetzt sind, längs der die Raststifte bzw. die Rastvertiefungen angeordnet sind.

Weitere vorteilhafte Ausgestaltungen der Erfindung sind Gegenstand der abhängigen Ansprüche sowie der folgenden Beschreibung.

Die Erfindung wird im Folgenden anhand der Figuren näher erläutert. Darin zeigen:
- Fig. 1: einen Haltearm nach der Erfindung;
- Fig. 2: ein Gelenk des Haltearms nach Figur 1;
- Fig. 3: das Gelenk nach Figur 2, wobei einer der Gelenkkörper weggelassen ist;
- Fig. 4: eine abgewickelte Querschnittsansicht des Gelenks;
- Fig. 5: den Schnitt A-A nach Figur 4 als geschnittene Draufsicht;
- Fig. 6: eine weitere Schnittansicht mit einem in einer Rastvertiefungen aufgenommenen Raststift;
- Fig. 7: den Schnitt B-B nach Figur 5; und
- Fig. 8: den Schnitt C-C nach Figur 5.

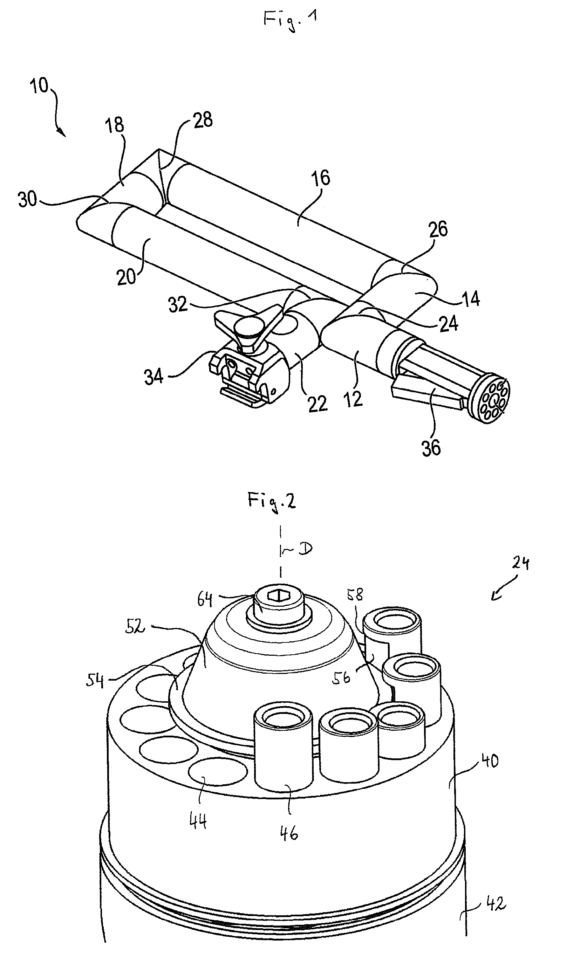

Figur 1 zeigt einen Haltearm 10, der mehrere starre Halteglieder 12, 14, 16, 18, 20 und 22 aufweist, die über Gelenke 24, 26, 28, 30 und 32 miteinander gekoppelt sind. An einem Ende des Haltearms 10 ist eine Befestigungsvorrichtung 34 angeordnet, die dazu dient, den Haltearm 10 an einer nicht gezeigten Gleitschiene eines OP-Tisches anzubringen. An dem anderen Ende des Haltearms 10 befindet sich ein Handgriff 36, der von dem Benutzer manuell betätigt werden kann, um den Haltearm 10 zu entriegeln.

Wird keine Betätigungskraft auf den Handgriff 36 ausgeübt, so sind die Halteglieder 12, 14, 16, 18, 20 und 22 des Haltearms 10 über die Gelenke 24, 26, 28, 30 und 32 starr miteinander gekoppelt. In diesem Zustand bildet der Haltearm 10 eine starre Einheit.

Drückt der Benutzer den Handgriff 36, so werden die über die Gelenke 24, 26, 28, 30 und 32 miteinander gekoppelten Halteglieder 12, 14, 16, 18, 20 und 22 über eine Entriegelungsmechanik gegeneinander beweglich, so dass der Benutzer den Haltearm 10 wie gewünscht im Raum ausrichten kann. Lässt der Benutzer anschließend den Haltegriff 36 wieder los, so werden die Gelenke 24, 26, 28, 30 und 32 verriegelt, und der Haltearm 10 wird in seiner veränderten Ausrichtung fixiert.

Anhand der Figuren 2 bis 8 werden im Folgenden Aufbau und Funktionsweise eines der baugleichen Gelenke 24, 26, 28, 30 und 32 erläutert. Beispielhaft wird hier auf das Gelenk 24 Bezug genommen.

Wie in Figur 2 gezeigt, weist das Gelenk 24 einen ersten Gelenkkörper 40 in Form eines Revolvers und einen zweiten Gelenkkörper 42 in Form einer Gelenkplatte auf. Dabei ist der erste Gelenkkörper 40 mit dem Halteglied 12 und der zweite Gelenkkörper 42 mit dem Halteglied 14 gekoppelt. Im verriegelten Zustand des Gelenks 24 sind die beiden Gelenkkörper 40 und 42 starr miteinander gekoppelt, während sie im entriegelten Zustand des Gelenks 24 um eine Drehachse D, die mit den Mittelachsen der beiden Gelenkkörper 40 und 42 zusammenfällt, gegeneinander verdrehbar.

Der Revolver 40 hat mehrere, vorzugsweise kreisförmig angeordnete Durchgangsbohrungen 44, in denen Raststifte 46 axial geführt sind. Im vorliegenden Ausführungsbeispiel sind zwölf Durchgangsbohrungen 44 und dementsprechend zwölf Raststifte 46 vorgesehen, wobei in den Figuren 2 bis 5 nur fünf der zwölf Raststifte 46 dargestellt sind.

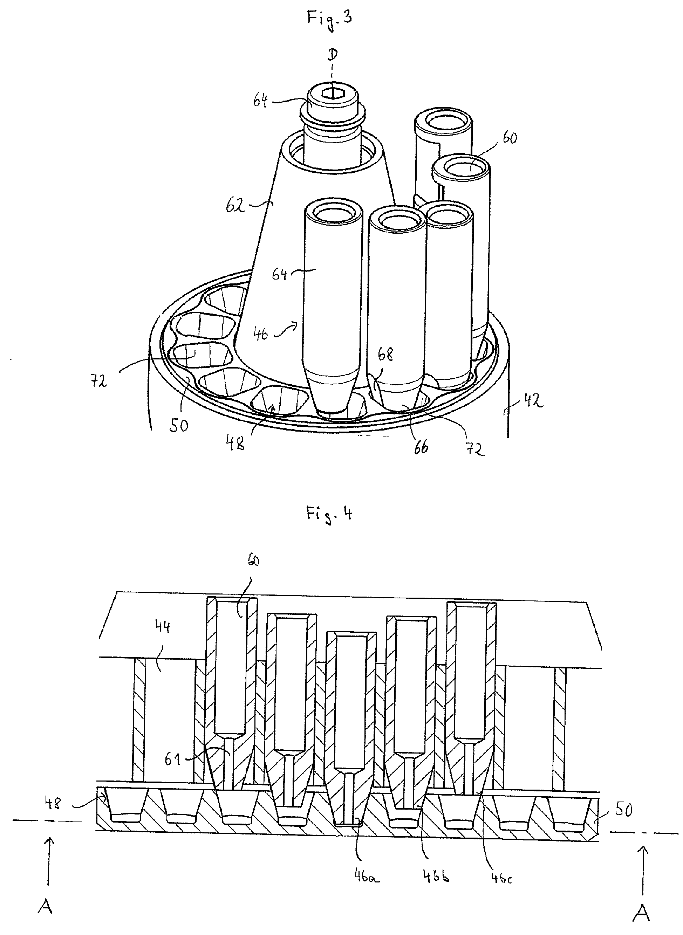

Wie der Darstellung nach Figur 3, in welcher der erste Gelenkkörper 40 weggelassen ist, zu entnehmen ist, weist der zweite Gelenkkörper 42 mehrere Rastvertiefungen 48 auf. Die Rastvertiefungen 48 sind in einer Rastscheibe 50 ausgebildet, die in dem zweiten Gelenkkörper 42 eingesetzt ist. Die Rastvertiefungen 48 dienen der Aufnahme der Raststifte 46, um die beiden Gelenkkörper 40 und 42 in nachfolgend beschriebener Weise zu verriegeln. Zu beachten ist hierbei, dass die Zahl an Rastvertiefungen 48 verschieden von der Zahl an Raststiften 46 ist. In dem vorliegenden Ausführungsbeispiel, in dem zwölf Raststifte 46 vorgesehen sind, weist der zweite Gelenkkörper 42 dreizehn Rastvertiefungen 48 auf.

Wie in Figur 2 gezeigt, ist auf der Oberseite des ersten Gelenkkörpers 40 ein glockenförmiges Hebeelement 52 montiert, das der Entriegelung des Gelenks 24 dient. Hierzu wirkt das Hebeelement 52 mit einem in den Figuren nicht gezeigten Entriegelungsgestänge zusammen, das mittels des Handgriffs 36 betätigbar ist, um das Hebeelement 52 längs der Drehachse D (in Figur 2 nach oben) zu verschieben. Das Hebeelement 52 weist an seinem unteren Rand einen Ringflansch 54 auf. Die Raststifte 46 haben jeweils eine dem Ringflansch 54 zugewandte Aussparung 56, in die der Ringflansch 54 greift. Durch die Aussparung 56 ist an dem jeweiligen Raststift 46 eine Stufe 58 ausgebildet, mit welcher der Ringflansch 54 (in Figur 2 von unten) in Anlage kommt, wenn das Hebeelement 52 mit Hilfe des Entriegelungsgestänges längs der Drehachse D angehoben wird. Wird das Hebeelement 52 weit genug angehoben, so führt es über seinen Ringflansch 54 die Raststifte 46 soweit mit sich, dass diese aus den Rastvertiefungen 48 gelöst werden. In diesem Zustand sind die beiden Gelenkkörper 40 und 42 um die Drehachse D gegeneinander beweglich.

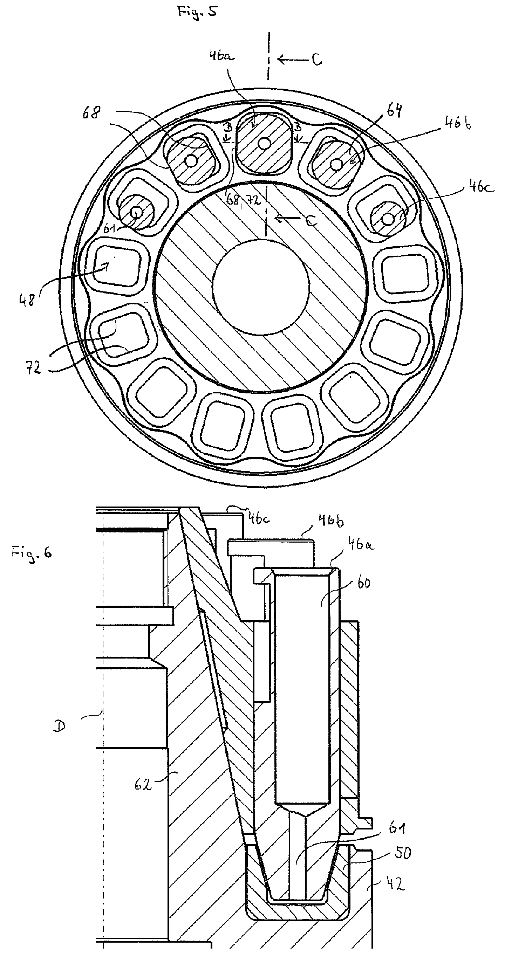

Die Raststifte 46 sind in den ihnen zugeordneten Durchgangsbohrungen 44 des ersten Gelenkkörpers 40 federnd gelagert. Hierzu ist jedem Raststift 46 ein in den Figuren nicht gezeigtes Vorspannelement zugeordnet, das in einer in dem Raststift 46 ausgebildeten Aufnahmebohrung 60 sitzt (vgl. Figuren 4 und 6). An die Aufnahmebohrung 60 schließt eine Entlüftungsbohrung 61 an.

Durch die Vorspannelemente sind die Raststifte 46 gegen den zweiten Gelenkkörper 42 vorgespannt. Befindet sich eine der Raststifte 46 im Bereich einer der Rastvertiefungen 44, so wird er auf diese Weise ganz oder teilweise in die Rastvertiefung 44 gedrückt. Das Hebeelement 42 löst also die Raststifte 46 entgegen dieser Vorspannung aus den Rastvertiefungen 44.

Der erste Gelenkkörper 40 ist auf einem in Figur 3 gezeigten Lagerkonus 62 gelagert. Über eine Befestigungsschraube 64 ist das Hebeelement 42 an einem nicht gezeigten Schubgestänge montiert. Das Hebeelement 52 sitzt über dem Lagerkonus 62.

Wie weiter oben erwähnt, ist die Zahl an Raststiften 46 verschieden von der Zahl an Rastvertiefungen 44. Somit ist der Winkelabstand zweier unmittelbar benachbarter Raststifte 46 verschieden von dem Winkelabstand zweier unmittelbar benachbarter Rastvertiefungen 48. Darauf ergibt sich eine noniusartige Zuordnung der Raststifte 46 zu den Rastvertiefungen 48. Diese noniusartige Zuordnung hat zufolge, dass in dem vorliegenden Ausführungsbeispiel im verriegelten Zustand des Gelenks 24 jeweils nur einer der Raststifte 46 vollständig in eine der Rastvertiefungen 48 eingetaucht ist, während die Raststifte 46, die diesem voll eingetauchten Raststift benachbart sind, sukzessive weniger tief in den ihnen zugeordneten Rastvertiefungen 48 aufgenommen sind. Die verbleibenden Raststifte 46 sitzen dagegen an der Oberseite der Rastscheibe 50 auf, ohne in eine der Rastvertiefungen 48 einzutauchen. Diese noniusartige Zuordnung zwischen den Raststiften 46 und den Rastvertiefungen 48 ist insbesondere auch der abgewickelten Darstellung nach Figur 4 und der teilweise geschnittenen Ansicht nach Figur 6 entnehmbar.

Die Raststifte 46 haben jeweils einen Stiftkörper 64 und einen daran anschließenden, axial verjüngten Eingriffsteil 66. Im vorliegenden Ausführungsbeispiel ist der Eingriffsteil 66 im Wesentlichen als Kegelstumpf geformt. Erfindungsgemäß sind auf diametral entgegengesetzten Seiten diesen kegelstumpfförmigen Eingriffsteils 66 zwei abgeflachte, vorzugsweise ebene Anlageflächen 68 ausgebildet, die sich jeweils über die gesamte Länge des Eingriffsteils 66 erstrecken. In der perspektivischen Darstellung nach Figur 3 ist jeweils nur eine dieser beiden Anlageflächen 68 gezeigt, während in der geschnittenen Darstellung nach Figur 5 jeweils beide Anlageflächen 68 angedeutet sind.

Die Rastvertiefungen 48 haben in dem vorliegenden Ausführungsbeispiel jeweils die Form eines Pyramidenstumpfs, der näherungsweise eine rechteckige Grundfläche aufweist. Infolge dieser pyramidenstumpfförmigen Ausbildung hat die jeweilige Rastvertiefung 48 zwei Seitenflächen 72, die den beiden Anlageflächen 68 des zugehörigen Raststifts 46 zugeordnet sind und mit diesen in Kontakt kommen, wenn der Raststift 46 in die Rastvertiefung 48 eintaucht. Die beiden vorstehend genannten Seitenflächen 72 der Rastvertiefung 48 bilden demnach Anlageflächen, die dazu bestimmt sind, mit den Anlageflächen 68 des Raststifts 46 in Kontakt zu kommen.

Die Art und Weise, wie die Raststifte 46 in Abhängigkeit ihrer Eintauchtiefe mit den Rastvertiefungen 48 erfindungsgemäß in flächigen Kontakt kommen, ist insbesondere in den Figuren 4 und 5 veranschaulicht. Dort ist derjenige Raststift, der vollständig in die zugehörige Rastvertiefung eintaucht, mit 46a bezeichnet, während die beiden ihm unmittelbar benachbarten Raststifte, die jeweils nur teilweise in die zugehörigen Rastvertiefungen eintauchen, mit 46b bezeichnet sind. Die in den Figuren 4 und 5 mit 46c bezeichneten Raststifte tauchen nur noch marginal in die zugehörigen Rastvertiefungen ein.

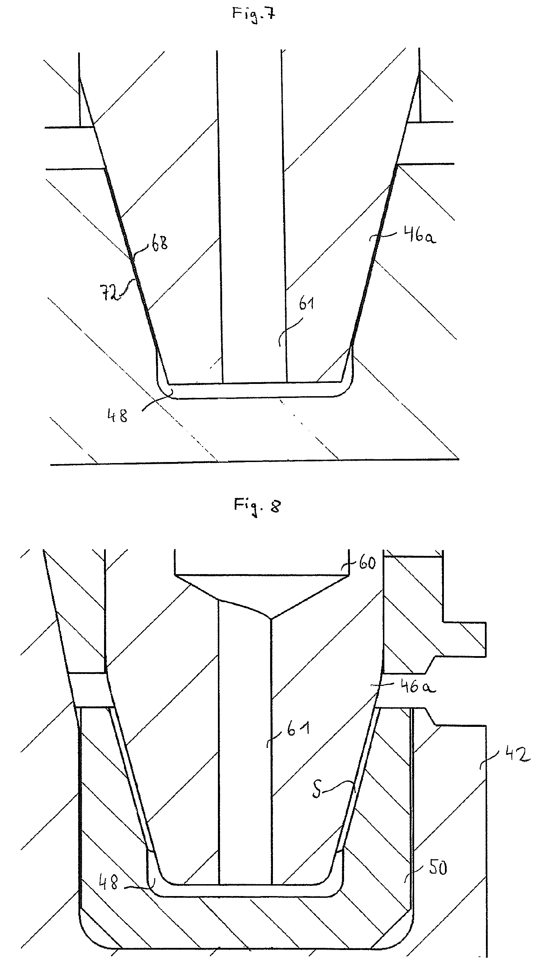

Wie in Figur 5 gezeigt, liegt der vollständig eintauchende Raststift 46a mit seinen beiden Anlageflächen 68 voll an den beiden Anlageflächen 72 der zugehörigen Rastvertiefung 48 an. Zu beachten ist hierbei, dass der Raststift 46a nur mit seinen beiden Anlageflächen 68 Kontakt mit der Rastvertiefung 48 hat, d.h. außerhalb dieser Anlageflächen 68 ein Spiel gegenüber den Seitenwänden der Rastvertiefung 48 hat. Dieser Sachverhalt ist nochmals in den Darstellungen nach Figur 7 und Figur 8 veranschaulicht. Dabei zeigt Figur 7 den Schnitt B-B und Figur 8 den Schnitt C-C nach Figur 5. Das Spiel zwischen dem Raststift 46a und der Rastvertiefung 48 ist in den Figuren 7 und 8 mit S bezeichnet. Die Figuren 7 und 8 zeigen auch, dass die Stirnfläche des vollständig eintauchenden Raststifts 46a einen geringfügigen Abstand von dem Boden der Rastvertiefung 48 aufweist, um ein Aufsetzen auf dem Boden zu vermeiden. Um diesbezüglich eine gewisse Fertigungstoleranz zu gewährleisten, läuft die Rastvertiefung 48 in unmittelbarer Nähe ihres Bodens auch nicht mehr axial zu.

Die den vollständig eingetauchten Raststift 46a unmittelbar benachbarten, nur teilweise eingetauchten Raststifte 46b liegen jeweils nur mit einer ihrer Anlageflächen 68 an der zugehörigen Anlagefläche 72 der jeweiligen Rastvertiefung 46 an. Abgesehen von dieser flächigen Anlage haben auch sie keinen Kontakt mit den Seitenwänden der ihnen zugeordneten Vertiefungen 48. Entsprechendes gilt für die Raststifte 46c, die nur noch sehr geringfügig in die zugehörigen Rastvertiefungen 48 eintauchen.

Wie in Figur 5 gezeigt, sind die ebenen Anlageflächen 68 der Raststifte 46 und die ebenen Anlageflächen 72 der Rastvertiefungen 48 so angeordnet, dass sie längs eines imaginären Kreises ausgerichtet sind, der die kreisförmige Anordnung der Raststifte 46 und der Rastvertiefungen 48 definiert. Entlang dieses Kreises werden die Raststifte 46 und die Rastvertiefungen 48 gegeneinander verdreht, wenn die beiden Gelenkkörper 40 und 42 gegeneinander bewegt werden. Die Flächenpressung, die zwischen den Anlageflächen 68 und 72 auftritt, wirkt demnach in Richtung der Drehbewegung der beiden Gelenkkörper 40 und 42.

Wie insbesondere aus der Darstellung nach Figur 5 hervorgeht, ist durch das erfindungsgemäße Vorsehen der abgeflachten Anlageflächen 68 und 72 dafür gesorgt, dass die Raststifte 46 für jede Eintauchtiefe einen flächigen Kontakt mit der zugehörigen Rastvertiefung 48 aufweisen. Insbesondere wird bei nicht vollständig eingetauchten Raststiften 46 der im Stand der Technik auftretende Linienkontakt vermieden und damit die Flächenpressung reduziert.

Da der vollständig eingetauchte Raststift 46a nur im Bereich seiner Anlageflächen 68 Kontakt mit der Rastvertiefung 48 hat, wird einem Verklemmen des Raststifts 46a entgegengewirkt. Dies gilt auch für die nur teilweise eintauchenden Raststifte 46b und 46c. So können auch statische Überbestimmungen, Toleranzschwankungen und Achsfehler aufgefangen werden.

### Bezugszeichenliste

- 10: Haltearm
- 12, 14, 16, 18, 20, 22: Halteglieder
- 24, 26, 28, 30, 32: Gelenke
- 36: Handgriff
- 40: erster Gelenkkörper
- 42: zweiter Gelenkkörper
- 44: Durchgangsbohrungen
- 46: Raststifte
- 48: Rastvertiefungen
- 50: Rastscheibe
- 52: Hebeelement
- 54: Ringflansch
- 56: Aussparung
- 58: Stufe
- 60: Aufnahmebohrung
- 61: Entlüftungsbohrung
- 62: Lagerkonus
- 64: Befestigungsschraube
- 66: Eingriffsteil
- 68, 72: Anlageflächen

## Patentansprüche

1. Medizinischer Haltearm, umfassend mindestens ein Gelenk (24, 26, 28, 30, 32) mit zwei Gelenkkörpern (40, 42), die um eine Drehachse (D) gegeneinander verdrehbar sind, wobei
ein erster der Gelenkkörper (40) mehrere Raststifte (46) und ein zweiter der Gelenkkörper (42) mehrere Rastvertiefungen (48) aufweist,
die Zahl an Raststiften (46) verschieden ist von der Zahl an Rastvertiefungen (48),
die Raststifte (46) jeweils einen axial verjüngten Eingriffsteil (66) aufweisen,
die Rastvertiefungen (48) jeweils zur wahlweisen, das Gelenk (24, 26, 28, 30, 32) verriegelnden Aufnahme jedes der Eingriffsteile (66) axial verjüngt ausgebildet sind, und
bei verriegeltem Gelenk (24, 26, 28, 30, 32) mindestens einer der Raststifte (46) mit seinem verjüngten Eingriffsteil (66) vollständig in einer der verjüngten Rastvertiefungen (48) aufgenommen ist, während mindestens einer der anderen Raststifte (46) mit seinem verjüngten Eingriffsteil (66) nur zum Teil in einer der anderen verjüngten Rastvertiefungen (48) aufgenommen ist,
**dadurch gekennzeichnet, dass**
der Eingriffsteil (66) des jeweiligen Raststifts (46) mindestens eine erste abgeflachte Anlagefläche (68) und die jeweilige Rastvertiefung (48) mindestens eine zweite abgeflachte Anlagefläche (72) hat, und
bei Aufnahme des Eingriffsteils (66) des jeweiligen Raststifts (46) in der jeweiligen Rastvertiefung (48) die erste und die zweite Anlagefläche (68, 72) zumindest zum Teil in flächigem Kontakt miteinander sind.

2. Haltearm (10) nach Anspruch 1, **dadurch gekennzeichnet, dass** die erste und die zweite Anlagefläche (68, 72) jeweils aus einer ebenen Fläche gebildet sind.

3. Haltearm (10) nach Anspruch 1 oder 2, **dadurch gekennzeichnet, dass** der Eingriffsteil (66) des jeweiligen Raststifts (46) als Kegelstumpf geformt ist, an dem die erste Anlagefläche (68) ausgebildet.

4. Haltearm (10) nach einem der vorhergehenden Ansprüche, **dadurch gekennzeichnet, dass** die jeweilige Rastvertiefung (48) als Pyramidenstumpf geformt ist und die zweite Anlagefläche (72) aus einer Seitenfläche der so geformten Rastvertiefung (48) gebildet ist.

5. Haltearm (10) nach einem der vorhergehenden Ansprüche, **dadurch gekennzeichnet, dass** die mindestens eine erste Anlagefläche (68) des jeweiligen Raststifts (46) mindestens zwei Anlageflächen beinhaltet, die auf diametral entgegengesetzten Seiten des Eingriffsteils (66) des Raststifts (46) angeordnet sind.

6. Haltearm (10) nach einem der vorhergehenden Ansprüche, **dadurch gekennzeichnet, dass** der jeweilige Raststift (46) bei Aufnahme seines Eingriffsteils (66) in der Rastvertiefung (48) nur mit seiner ersten Anlagefläche (68) Kontakt mit der Rastvertiefung (48) hat.

7. Haltearm (10) nach einem der vorhergehenden Ansprüche, **dadurch gekennzeichnet, dass** die Raststifte (46) und die Rastvertiefungen (48) jeweils eine kreisförmige Anordnung bilden.

8. Haltearm (10) nach Anspruch 7, **dadurch gekennzeichnet, dass** die ersten Anlageflächen (68) der Raststifte (46) und die zweiten Anlageflächen (72) der Rastvertiefungen (48) jeweils längs der kreisförmigen Anordnung ausgerichtet sind.

9. Haltearm (10) nach einem der vorhergehenden Ansprüche, **dadurch gekennzeichnet, dass** der erste Gelenkkörper (40) mehrere Durchgangsbohrungen (44) hat, in denen jeweils einer der Raststifte (46) axial geführt ist.

10. Haltearm (10) nach einem der vorhergehenden Ansprüche, **dadurch gekennzeichnet, dass** die Raststifte (46) an dem ersten Gelenkkörper (40) federnd gelagert und gegen den zweiten Gelenkkörper (42) vorgespannt sind.

11. Haltearm (10) nach einem der vorhergehenden Ansprüche, **dadurch gekennzeichnet, dass** die Zahl an Rastvertiefungen (48) größer als die Zahl an Raststiften (46) ist.

12. Haltearm (10) nach einem der vorhergehenden Ansprüche, **gekennzeichnet durch** mindestens zwei starre Halteglieder (12, 14, 16, 18, 20, 22), von denen ein erstes Halteglied mit dem ersten Gelenkkörper (40) und das zweite Halteglied mit dem zweiten Gelenkkörper (42) gekoppelt ist.

## Claims

1. A medical holding arm comprising at least one joint (24, 26, 28, 30, 32) with two joint bodies (40, 42) which are rotatable relative to each other about a rotational axis (D), wherein
a first of the joint bodies (40) has a plurality of locking pins (46) and a second of the joint bodies (42) has a plurality of locking recesses (48),
the number of locking pins (46) differs from the number of locking recesses (48),
the locking pins (46) each have an axially tapered engagement part (66),
the locking recesses (48) are respectively formed axially tapered to selectively receive each of the engagement parts (66) for locking the joint (24, 26, 28, 30, 32), and,
when the joint (24, 26, 28, 30, 32) is locked, at least one of the locking pins (46) is received with its tapered engagement part (66) completely in one of the tapered locking recesses (48), while at least one of the other locking pins (46) is received with its tapered engagement part (66) only partially in one of the other tapered locking recesses (48),
**characterized in that**
the engagement part (66) of the respective locking pin (46) has at least one first flattened contact surface (68), and the respective locking recess (48) has at least one second flattened contact surface (72), and,
when the engagement part (66) of the respective locking pin (46) is received in the respective locking recess (48), the first and second contact surface (68, 72) are at least partially in flat contact with each other.

2. The holding arm (10) according to claim 1, **characterized in that** the first and second contact surfaces (68, 72) are respectively formed from a flat surface.

3. The holding arm (10) according to claim 1 or 2, **characterized in that** the engagement part (66) of the respective locking pin (46) is formed as a truncated cone on which the first contact surface (68) is formed.

4. The holding arm (10) according to any of the preceding claims, **characterized in that** the respective locking recess (48) is formed as a truncated pyramid and the second contact surface (72) is formed from a side surface of the locking recess (48) formed in this manner.

5. The holding arm (10) according to any of the preceding claims, **characterized in that** the at least one first contact surface (68) of the respective locking pin (46) comprises at least two contact surfaces, which are arranged on diametrically opposed sides of the engagement part (66) of the locking pin (46).

6. The holding arm (10) according to any of the preceding claims, **characterized in that** the respective locking pin (46), when its engagement part (66) is received in the locking recess (48), contacts the locking recess (48) only with its first contact surface (68).

7. The holding arm (10) according to any of the preceding claims, **characterized in that** the locking pins (46) and the locking recesses (48) each form a circular arrangement.

8. The holding arm (10) according to claim 7, **characterized in that** the first contact surfaces (68) of the locking pins (46) and the second contact surfaces (72) of the locking recesses (48) are each aligned along the circular arrangement.

9. The holding arm (10) according to any of the preceding claims, **characterized in that** the first joint body (40) has a plurality of through bores (44), in each of which one of the locking pins (46) is axially guided.

10. The holding arm (10) according to any of the preceding claims, **characterized in that** the locking pins (46) are resiliently supported on the first joint body (40) and are biased against the second joint body (42).

11. The holding arm (10) according to any one of the preceding claims, **characterized in that** the number of locking recesses (48) is greater than the number of locking pins (46).

12. The holding arm (10) according to any of the preceding claims, **characterized by** at least two rigid holding elements (12, 14, 16, 18, 20, 22), of which a first holding element is coupled to the first joint body (40) and the second holding element is coupled to the second joint body (42).

## Revendications

1. Bras de support médical comprenant au moins une articulation (24, 26, 28, 30, 32) avec deux corps d'articulation (40, 42), qui peuvent tourner l'un par rapport à l'autre autour d'un axe de rotation (D), dans lequel
un premier des corps d'articulation (40) présente plusieurs broches d'encliquetage (46) et un second des corps d'articulation (42) plusieurs cavités d'encliquetage (48),
le nombre de broches d'encliquetage (46) est différent du nombre de cavités d'encliquetage (48),
les broches d'encliquetage (46) présentent respectivement une partie d'engagement (66) réduite axialement,
les cavités d'encliquetage (48) sont réalisées réduites axialement pour recevoir au choix chacune des parties d'engagement (66) en verrouillant l'articulation (24, 26, 28, 30, 32), et
lorsque l'articulation (24, 26, 28, 30, 32) est verrouillée, au moins l'une des broches d'encliquetage (46) est reçue avec sa partie d'engagement (66) réduite complètement dans l'une des cavités d'encliquetage (48) réduites, tandis qu'au moins l'une des autres broches d'encliquetage (46) n'est reçue avec sa partie d'engagement (66) réduite qu'en partie dans l'une des autres cavités d'encliquetage (48) réduites,
**caractérisé en ce que**
la partie d'engagement (66) de la broche d'encliquetage (46) respective a au moins une première surface d'appui (68) aplatie et la cavité d'encliquetage (48) respective au moins une seconde surface d'appui (72) aplatie, et
lors de la réception de la partie d'engagement (66) de la broche d'encliquetage (46) respective dans la cavité d'encliquetage (48) respective, la première et la seconde surface d'appui (68, 72) sont au moins en partie en contact à plat l'une avec l'autre.

2. Bras de support (10) selon la revendication 1, **caractérisé en ce que** la première et la seconde surface d'appui (68, 72) sont respectivement formées d'une surface lisse.

3. Bras de support (10) selon la revendication 1 ou 2, **caractérisé en ce que** la partie d'engagement (66) de la broche d'encliquetage (46) respective est formée en tronc de cône, au niveau duquel la première surface d'appui (68) est réalisée.

4. Bras de support (10) selon l'une quelconque des revendications précédentes, **caractérisé en ce que** la cavité d'encliquetage (48) respective est formée en tronc de pyramide et la seconde surface d'appui (72) est formée d'une surface latérale de la cavité d'encliquetage (48) ainsi formée.

5. Bras de support (10) selon l'une quelconque des revendications précédentes, **caractérisé en ce que** l'au moins une première surface d'appui (68) de la broche d'encliquetage (46) respective comprend au moins deux surfaces d'appui qui sont agencées sur des côtés diamétralement opposés de la partie d'engagement (66) de la broche d'encliquetage (46).

6. Bras de support (10) selon l'une quelconque des revendications précédentes, **caractérisé en ce que** la broche d'encliquetage (46) respective a, lors de la réception de sa partie d'engagement (66) dans la cavité d'encliquetage (48), un contact avec la cavité d'encliquetage (48) uniquement avec sa première surface d'appui (68).

7. Bras de support (10) selon l'une quelconque des revendications précédentes, **caractérisé en ce que** les broches d'encliquetage (46) et les cavités d'encliquetage (48) forment respectivement un agencement circulaire.

8. Bras de support (10) selon la revendication 7, **caractérisé en ce que** les premières surfaces d'appui (68) des broches d'encliquetage (46) et les secondes surfaces d'appui (72) des cavités d'encliquetage (48) sont orientées respectivement le long de l'agencement circulaire.

9. Bras de support (10) selon l'une quelconque des revendications précédentes, **caractérisé en ce que** le premier corps d'articulation (40) a plusieurs alésages de passage (44) dans lesquels respectivement l'une des broches d'encliquetage (46) est guidée axialement.

10. Bras de support (10) selon l'une quelconque des revendications précédentes, **caractérisé en ce que** les broches d'encliquetage (46) sont montées de manière élastique au niveau du premier corps d'articulation (40) et sont précontraintes contre le second corps d'articulation (42).

11. Bras de support (10) selon l'une quelconque des revendications précédentes, **caractérisé en ce que** le nombre de cavités d'encliquetage (48) est plus grand que le nombre de broches d'encliquetage (46).

12. Bras de support (10) selon l'une quelconque des revendications précédentes, **caractérisé par** au moins deux éléments de retenue rigides (12, 14, 16, 18, 20, 22) dont un premier élément de retenue est couplé au premier corps d'articulation (40) et le second élément de retenue au second corps d'articulation (42).
